# EUROPEAN PATENT APPLICATION

(11) **EP 4 099 362 A2**
(43) Date of publication of application: **07.12.2022**
(21) Application number: 22175613.3
(22) Date of filing: 26.05.2022
(51) Int. Cl.: H01J 49/00, G01N 33/50, G16B 20/00

(54) **MASS SPECTROMETER UTILIZING MASS SPECTRAL DATABASE SEARCH FOR COMPOUND IDENTIFICATION**

(30) Priority: 01.06.2021 US 202163195519 P
(71) Applicant: Thermo Finnigan LLC, San Jose, CA 95134 (US)
(72) Inventor: ZABROUSKOV, Vladimir, Belmont, 94002 (US); BARSHOP, William Dana, Sunnyvale (US); CANTERBURY, Jesse, Shoreline, 98177 (US); SHARMA, Seema, San Jose, 95130 (US)
(74) Representative: Boult Wade Tennant LLP

(57) **Abstract**

Real-time search (RTS) for mass spectrometry is described. In one instance, precursor ions generated from a sample are introduced into a mass spectrometer during an introduction period. The precursor ions are fragmented to form product ions, and a mass spectrum is acquired. During the introduction period, scores indicating the similarity between the mass spectrum and a candidate mass spectrum are identified. Based on the distribution of the scores, an action is performed.

## Description

### TECHNICAL FIELD

This disclosure relates to apparatus and methods for mass spectrometry, and more particularly to operation of a mass spectrometer for identifying unknown compounds in a sample.

### BACKGROUND

A current focus of biological mass spectrometry is the identification, quantification, and structural elucidation of molecules. Metabolites are often small biological molecules that are an intermediate or end product of metabolism. In the context of mass spectrometry and metabolomics, a compound such as a metabolite can be identified in a mixture by performing controlled fragmentation of certain ions (referred to as tandem or MSn mass spectrometry) to yield product ions, whose mass spectra provides information that may be highly useful to confirm identification, determine a quantity, or derive structural details regarding the metabolic compound of interest.

However, unknown or unpredicted metabolites are challenging to identify in a complex mixture. Multiple experiments are performed with post-acquisition data processing, and manual adjustment of experiment method setup is performed between the experiments in response to the data processing. This takes a significant amount of time, and often misses unknown or unpredicted metabolites from the analysis.

### SUMMARY

In accordance with one aspect of the present invention, an embodiment of a method of operating a mass spectrometer to analyze a metabolite of a unmetabolized compound is described. The method includes introducing precursor ions generated from a sample into the mass spectrometer during an introduction period, fragmenting the precursor ions to form product ions, and acquiring a first mass spectrum representative of the product ions. The method further includes generating, during the introduction period, scores indicative of a similarity between the first mass spectrum and a candidate mass spectrum of a candidate compound, the candidate mass spectrum stored in a mass spectra database, the scores indicating that the first mass spectrum represents a metabolite of the candidate compound and performing an action on the precursor ions or the product ions based on a distribution of the scores.

In another aspect of the present invention, an embodiment of a method of performing mass spectrometry includes introducing precursor ions generated from a sample into the mass spectrometer during an introduction period and fragmenting the precursor ions to form product ions. The embodiment further includes acquiring a first mass spectrum representative of the product ions, generating, during the introduction period, scores indicative of a similarity between the first mass spectrum and a candidate mass spectrum of a candidate compound, and performing an action on the precursor ions or the product ions based on a distribution of the scores.

In yet another aspect of the present invention, an embodiment of a mass spectrometry system operable to analyze a metabolite of a unmetabolized compound is described. The embodiment includes an ion source that introduces precursor ions generated from a sample into the mass spectrometry system during an introduction period. A mass analyzer separates the ions by mass-to-charge ratio and detector detects the separated ions from the mass analyzer and generating a representative signal. A controller generates a first mass spectrum representative of the ions based on the representative signal from the detector and generating, during the introduction period, scores indicative of a similarity between the first mass spectrum and a candidate mass spectrum of a candidate compound.

In fourth aspect of the present invention an embodiment of a mass spectrometry system operable to analyze a metabolite of a unmetabolized compound is described that includes a controller. A computer program product including one or more non-transitory computer-readable media having computer program instructions stored therein that are configured such that, when executed by the controller, the computer program instructions cause the mass spectrometry system to perform a set of acts. The set of acts includes introducing precursor ions generated from a sample into the mass spectrometer during an introduction period and fragmenting the precursor ions to form product ions. The set of acts further includes acquiring a first mass spectrum representative of the product ions, generating, during the introduction period, scores indicative of a similarity between the first mass spectrum and a candidate mass spectrum of a candidate compound, and performing an action on the precursor ions or the product ions based on a distribution of the scores.

### BRIEF DESCRIPTION OF THE DRAWINGS

Figure 1 illustrates an example of a mass spectrometer.
Figure 2 illustrates an example of a block diagram for operating a mass spectrometer such as the example of Figure 1 to acquire product ion mass spectra.
Figure 3 illustrates an example of a block diagram for determining similarity scores of an experimental mass spectrum.
Figure 4 illustrates an example of determining similarity scores of an experimental mass spectrum.
Figure 5 illustrates an example of a block diagram for operating a mass spectrometer to identify an unknown metabolite.
Figure 6 illustrates an example of an electronic device which may be used to implement some of the examples.

### DETAILED DESCRIPTION

Some of the material described in this disclosure includes mass spectrometers and techniques for real-time searching (RTS) in a Data Dependent Acquisition (DDA) methodology to detect compounds of interest in a mixture. In one example, an experimental mass spectrum is identified as representing a metabolite by identifying, via a mass spectra database, a similarity to a candidate mass spectrum representing the compound from which the metabolite was produced. Upon the identification of the similarity, an additional operation is performed, for example, MS3.

In this example, a sample for analysis includes metabolites that are the result of the metabolic breakdown of a pharmaceutical product, food, fluids, or other material. While some of the metabolites (or metabolic compounds) might be known, other metabolites might be unknown. Identification and quantification of these unknown metabolites is a significant interest in the field of metabolomics.

The mixture including the unknown metabolites (and other compounds) is introduced into a chromatography system such that different compounds in the mixture are separated and introduced into a mass spectrometer for analysis at different times in a single experimental run. The introduction period of a chromatographically separated compound into the mass spectrometer (i.e., the time between when the compound begins to elute from the chromatographic column and is delivered to the mass spectrometer inlet, and when elution is completed) is determined by the chromatographic peak width and defines the time available to perform mass spectrometry operations on the compound.

The analysis can include ionization of the compound to form precursor ions, which are subsequently fragmented to form product ions to acquire an experimental mass spectrum (i.e., MS2 or MS/MS). As described later in this disclosure, the experimental mass spectrum is then used to search a mass spectral database to identify a similar, but different, candidate mass spectrum representing the material that was metabolized and thereby formed the metabolite. That is, in one example, the experimental mass spectrum might represent an unknown metabolite that is similar, but not an exact match, to a pharmaceutical compound that was ingested and metabolized to form the unknown metabolite. The unknown metabolite represents a modified form of the pharmaceutical compound (e.g., includes a chemical modification) and, therefore, is represented by a slightly different mass spectrum than the pharmaceutical compound.

The similarity can be based on different scoring methodologies, and the distribution of scores using the different methodologies can result in performing different operations of the mass spectrometer. For example, if an experimental mass spectrum is similar to a candidate mass spectrum identified in the mass spectral database, and has a high cosine score but a low confidence score from two different scoring methodologies, then this might be indicative of an under fragmented MS2 scan. Accordingly, the MS2 scan might be performed a second time to obtain a better experimental mass spectrum for further analysis. In another example, MS3 might be performed on product ions, resulting in MS3 operated on metabolites while not performing MS3 on compounds that are not identified as metabolites.

These operations occur during the introduction time, or intake time, of the compound. As a result, the same sample that is chromatographically separated in a single injection into the mass spectrometer is analyzed with many MS2 scans to form product ion mass spectra as the experimental mass spectra, and those product ion mass spectra are used to search the mass spectral database multiple times during the introduction times of different compounds. A variety of rules are applied in real-time during the introduction time in a DDA analysis. This results in different operations performed by the mass spectrometer in response to the different distribution of scores. Thus, data processing occurs in real-time while the sample is analyzed (i.e., separated, eluted, ionized, etc.), and manual adjustment of experiment setup is avoided. Moreover, more unknown compounds can be identified.

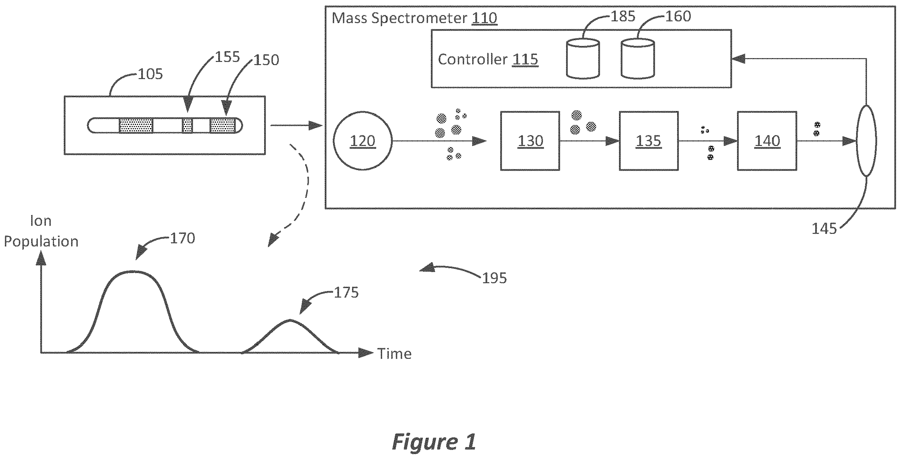

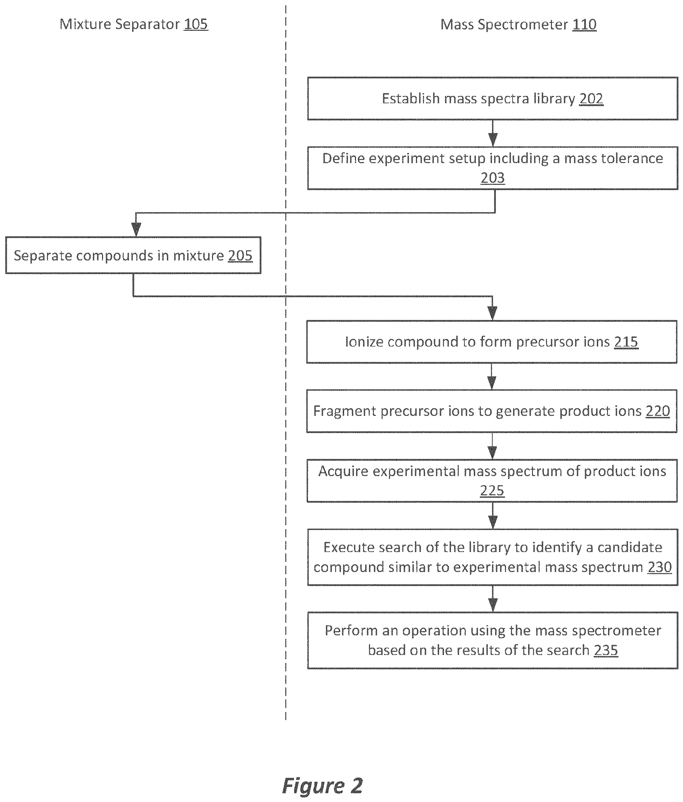

In more detail, Figure 1 illustrates an example of a mass spectrometer. Figure 2 illustrates an example of a block diagram for operating a mass spectrometer such as the example of Figure 1 to acquire product ion mass spectra. In the block diagram of Figure 2, a mass spectra library is established (202) and an experiment setup including a mass tolerance is defined (203). For example, in Figure 1, mass spectrometer 110 includes controller 115, which is a circuit configured to control the operation of mass spectrometer 110.

Controller 115 includes, or has access to, mass spectra library 185 and experiment setup 160. Mass spectra library 185 stores mass spectra to be used in a real-time search, for example, as an electronically-stored collection of information that includes one or both of: (i) data, such as amino acid or other molecular sequences for compounds, that may be employed to generate theoretical mass spectra based on predetermined rules (e.g., proteolysis cleavages, fragmentation predictions, etc.), or (ii) empirically derived spectra acquired previously for identified compounds (i.e., a spectral library), though other types of information related to compounds can also be stored. The theoretical or empirically-derived mass spectra contained in or derived from the mass spectral database includes a list of ion m/z's and optionally the corresponding measured or predicted intensities. In the example of metabolomics, the mass spectra might include MS2 mass spectra of a pharmaceutical compound (or other unmetabolized compound) of interest for which unknown metabolites are desired to be identified.

Experiment setup 160 includes details on how to operate mass spectrometer 110. For example, the user might define specific operational parameters of the mass spectrometer for an experimental run for an injection of a sample, including selecting a mass tolerance to define experimental mass spectra that can be considered similar fragmentation patterns of the unmetabolized compound. Thus, the mass tolerance defines how much of a difference in the properties of the experimental mass spectrum are allowed for the experimental mass spectrum to be considered a metabolite of a compound represented by a candidate mass spectrum in mass spectra library 185. In some implementations, the mass tolerance is defined as wider than in an experiment identifying peptides due to looking for a similarity rather than a match between spectra. However, the mass tolerance can be wide as some metabolites can be very far away (in terms of m/z) from the original compound from which they formed.

Returning to Figure 2, once the mass spectral library and experiment is set up, the mixture, or sample, including the unknown metabolized compounds are provided to a mixture separator to separate the compounds in the mixture (205). For example, in Figure 1, mixture separator 105 can be a liquid chromatography (LC), gas chromatography (GC), capillary electrophoresis (CE), or other type of system used to separate compounds of a mixture. In the metabolomics example, the separate components of the mixture include unknown metabolites of the unmetabolized compound.

In Figure 1, mixture separator 105 is depicted as separating a mixture into several components including compounds 150 and 155. Compounds 150 and 155 are separated in space, or position, along a flow path (for example, within a chromatographic column) such that compounds 150 and 155 are introduced into mass spectrometer 110 at different times. This is depicted with chromatogram 195 showing peaks 170 and 175 representing compounds 150 and 155, respectively. Different compounds can have a different abundance within the mixture and, therefore, each of the peaks appears differently.

Returning to the block diagram of Figure 2, the compound can then be provided to the mass spectrometer and the compound can be ionized to form precursor ions (215). For example, in Figure 1, when compound 150 is introduced into mass spectrometer 110, it is first provided to ion source 120. Ion source 120 can ionize a material under analysis by removing or adding charge-carrying entities (e.g., hydrogen nuclei or electrons) to or from the material to provide the material with a positive or negative charge. This results in precursor ions forming from the ionization of compound 150. Ion source 120 will typically be of the electrospray ionization (ESI) type, but may instead utilize any other suitable ionization technique, including atmospheric-pressure chemical ionization (APCI) or atmospheric pressure photoionization (APPI).

Next, the precursor ions are mass isolated and then fragmented to generate product ions (220). For example, in Figure 1, the precursor ions of the compound formed by ion source 120 are transported via appropriate ion optics to mass selector 130. Mass selector 130 may take the form, in one example, of a quadrupole mass filter in which the amplitudes of the radio-frequency (RF) and resolving direct current (DC) voltages are adjusted such that only ions within a narrow range of m/z values are transmitted. Alternatively, mass selector 130 may be any suitable device capable of isolating ions within a m/z window of interest, such as an analytical ion trap or time-of-flight (TOF) mass analyzer. As depicted in Figure 1, some of the precursor ions of the compound are thus mass selected and passed onwards to fragmentation cell 135. In other words, some of the precursor ion species are mass isolated.

The precursor ions are then fragmented to generate the product ions. For example, in Figure 1, fragmentation cell 135 receives the precursor ions from mass analyzer 130 and fragments, or breaks up, the precursor ions into smaller product ions. Fragmentation is often performed on larger molecules, such as peptides or other biological molecules including metabolites, to allow for more detailed understanding of the structural composition. Fragmentation cell 135 can be implemented using many different types of disassociation techniques including collision-induced disassociation (CID), surface-induced dissociation (SID), electron-capture dissociation (ECD), electron-transfer dissociation (ETD), negative electron-transfer dissociation (NETD), electron-detachment dissociation (EDD), photodissociation, higher-energy C-trap dissociation (HCD), etc.

Next, in Figure 2, the product ions are subjected to mass analysis to acquire an experimental mass spectrum (225). For example, in Figure 1, the product ions formed by fragmentation cell 135 are provided to a mass analyzer 140, mass separated, and then provided to detector 145. Mass analyzer 140 can be any suitable device for separating ions according to their mass-to-charge ratios, including (without limitation) an orbital electrostatic trap, analytical quadrupole ion trap, Fourier Transform- Ion Cyclotron Resonance (FT-ICR) analyzer, TOF mass analyzer, or a quadrupole mass filter. Detector 145 can detect induced charge or current produced when the product ions provided by mass analyzer 140 pass by or hit a surface of detector 145. Thus, detector 145 generates signals representative of the m/z of the product ions. These signals are provided to controller 115, which can then generate an experimental mass spectrum using the detected signals.

In Figure 1, mass spectrometer 110 is a tandem mass spectrometer configured to implement a single stage of mass isolation and fragmentation, as indicated with the arrangement of fragmentation cell 135 between mass selector 130 and mass analyzer 140. This is often referred to as MS/MS or MS2 mass spectrometry. In certain implementations, it may be desirable to perform further stages of isolation and fragmentation of generations of product ions (e.g., MS3, MS4, MS5, etc. wherein n is a positive integer). In such cases, the components of mass spectrometer 110 may be configured to cause additional isolation/fragmentation operations. For example, mass analyzer 140 may be an analytical quadrupole ion trap mass analyzer that, in addition to performing the mass separation function for acquiring mass spectra, is also capable of executing steps of mass isolation and fragmentation.

Next, a search of the mass spectra library is performed to identify a candidate compound having a mass spectrum similar to the experimental mass spectrum (230). For example, in Figure 1, controller 115 analyzes the experimental mass spectrum generated using the signals provided by detector 145. As discussed in further detail below, controller 115 may be programmed to search the mass spectra database 185 to determine which, if any, of the candidate compounds contained in the database have (theoretical or empirically determined) mass spectra that are similar to the experimentally acquired mass spectrum, thereby establishing, to a reasonable degree of confidence, that the sample component that produced the experimental mass spectrum is a metabolite of the unmetabolized compound represented by the mass spectra in the mass spectra database 185.

Similar mass spectra can be determined using the mass tolerance defined in experiment setup 160. That is, the mass tolerance established by the user when setting up the experiment allows for how much of a difference between the experimental mass spectrum and the candidate compound mass spectrum in the mass spectral library is allowed for the two to be determined to be similar and, therefore, the compound that is ionized is a possible unknown metabolite. Thus, a different, but similar, mass spectrum is identified. In some implementations, a similarity score is calculated to determine the similarity between different mass spectra.

If the compound is not identified as being similar then additional operations performed by the mass spectrometer to further analyze the compound can be avoided. An experimental mass spectrum from a compound under analysis may not result in a similar mass spectrum identified in the mass spectra database because the experimental mass spectrum might be the same as one in the database (i.e., the compound is the same as the unmetabolized compound) or it is outside of the allowed mass tolerance range, meaning that the experimental mass spectrum is too different to be considered a metabolite. Thus, in terms of a similarity range (or scale, or score, etc.), too much similarity (e.g., a high similarity score) would be a match and therefore might represent the unmetabolized compound, and not enough similarity (e.g., a low similarity score) would represent a compound that is likely not a metabolite. However, a similarity in a range between the two aforementioned similarity ranges can represent a metabolite.

However, if a similarity is identified, then an additional operation using the mass spectrometer is performed (235). The additional operations that can be performed include performing MS2 again on the precursor ions, performing MS3 on the product ions (i.e., perform a second-generation product ion scan), fragmenting the precursor ions or the product ions at different activation energies to adjust how the fragmentation is done using the same fragmentation type (e.g., when using CID), fragmenting the precursor ions or the product ions using different fragmentation types (e.g., switching from CID to photodissocation), or performing any of the MSn scans using higher resolution on the precursor ions or the product ions. The MSn scans can include MS and selected ion monitoring (SIM) scans.

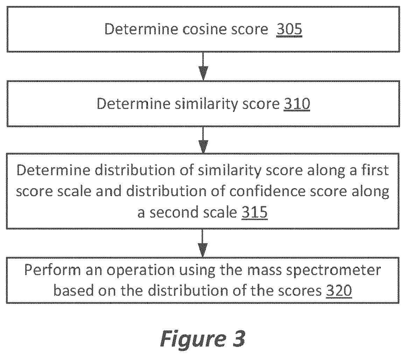

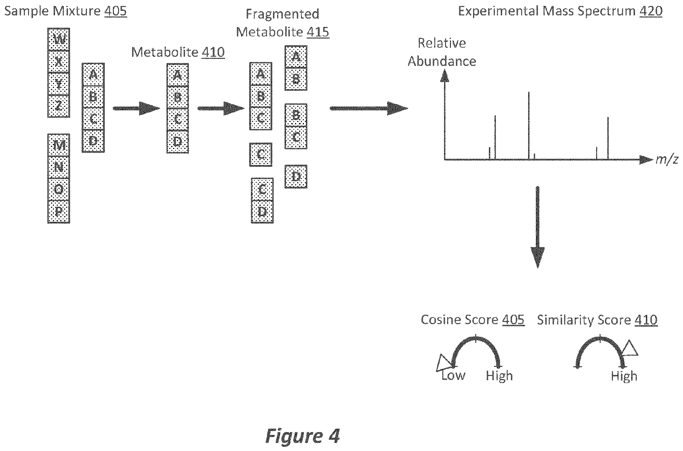

The identification of a similarity can be based on different scoring methodologies used with the mass tolerances identified in the experiment setup, and the distribution of scores using the different methodologies results in performing different operations of the mass spectrometer. Figure 3 illustrates an example of a block diagram for determining similarity scores of an experimental mass spectrum. Figure 4 illustrates an example of determining similarity scores of an experimental mass spectrum.

In Figure 4, sample mixture 405 includes three compounds, with metabolite 410 eluting to a mass spectrometer for ionization and analysis following chromatographic separation. Metabolite 410 is isolated and fragmented, forming fragmented metabolite 415 with a variety of product ions that are detected and used to generate experimental mass spectrum 420, which is an MS2 mass spectrum, or product ion mass spectrum. Experimental mass spectrum is then compared with all or some of the mass spectra indicated in the mass spectra library, and similarities are identified using scores including cosine score 405 and similarity score 410. If the scores are within a tolerance range, then a similarity is identified.

Cosine score 405 indicates a similarity of m/z values of fragments and relative intensities or abundances between the two mass spectra being compared. Similarity score 410 (or a confidence score) is a probabilistic calculation regarding a confidence of the accuracy of the identification, which is based on cosine score 405, number of matching peaks between the experimental mass spectrum and the mass spectrum it is being compared against in the mass spectra database, and other inputs. Together, the two scores provide different pieces of information regarding the quality of the similarity match between the experimental mass spectrum and the mass spectrum in the mass spectra database. Other types of scores, such as delta scores for the cosine score (e.g., to identify the difference between the highest score and the second-highest score of possible candidates), or a delta score for the similarity score can also be utilized.

Thus, in Figure 3, the cosine similarity score is determined (305) and the similarity score is determined (310). Next, the distribution of similarity scores is determined (315). For example, in Figure 4, cosine score 405 is a score along a spectrum or range of scores, visualized as low-to-high. Likewise, similarity score 410 is a score along a spectrum. Thus, these two scores are distributed along different scoring scales. In Figure 4, cosine score 405 is relatively low, and similarity score 410 is relatively high. Scores distributed in this manner might result in a specific operation to be performed by the mass spectrometer (320). The specific distribution of scores and the resulting actions can vary from experiment to experiment, and may be established during the experiment setup.

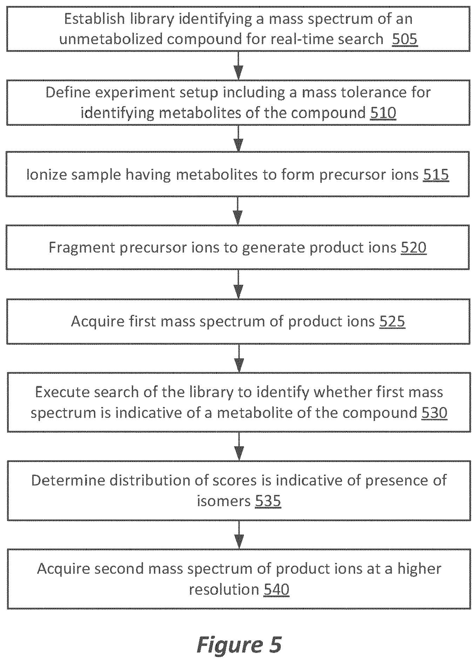

One example of a specific operation to perform based on the distribution of the scores is related to detection of isomers of metabolites, as demonstrated in Figure 5. In Figure 5, a mass spectra library is established, including identifying the mass spectrum of an unmetabolized compound (505). As previously discussed, the mass tolerance is defined (510), the sample is ionized to form precursor ions (515), the precursor ions are fragmented (520), and a first mass spectrum of product ions is acquired (525).

Next, the mass spectra library is searched to identify whether the first mass spectrum is indicative of a metabolite of the compound (530). This is based on the scores and the mass tolerance leading to a determination that the first mass spectrum might be different than a mass spectrum in the mass spectra library (also referred to here as a candidate mass spectrum), but close enough in similarity to be a metabolite.

The distribution of the scores is then determined, and the distribution is indicative of the presence of isomers (535) and a second mass spectrum of product ions at a higher resolution is acquired (540). Additionally, an MS3 operation on the product ions might be performed to determine further structural information of the isomers to distinguish them from each other.

These additional mass spectrometry operations might be performed because isomers are compounds with identical molecular formulas, but have different molecular structures. Thus, performing additional operations using the mass spectrometer might aid in differentiating between isomers. In one example, if the cosine score is in a high range, but the similarity score is in a low range, then this distribution of scores can be indicative of the presence of isomers.

Other examples of distribution of scores and mass spectrometer operations that can be performed based on the distribution of scores includes performing MS2 again in the presence of a high cosine and a low similarity score because this might be indicative of under fragmentation of the precursor ions. The additional MS2 can also be performed by altering the collision energy (e.g., increasing or decreasing the collision energy) used in the fragmentation, for example, when using CID. Many different distributions of the scores can result in different operations, meaning that the ranges of the scores can be divided into sub-ranges to provide many actions to be performed.

Many other operations can be performed upon establishment of a similarity between the experimental mass spectrum and the candidate mass spectrum. For example, if a similarity is identified, the DDA methodology can further identify whether the product ions are common to a compound class indicated in the library. If the product ions are not common to the compound class, then MS3 (or MSn) can be performed on the product ions. For example, in the analysis of fentanyl, product ions having m/z of 188 are commonly encountered. Because these product ions are commonly expected, MS3 might be refrained from being performed on them.

Another operation might be to refrain from performing MSn if a similarity is established. Rather, in this scenario, more operations (e.g., using different energies, different fragmentation techniques, or other techniques described herein) can be performed on the precursor ion to determine if a match can be established .

Other operations can include, upon determining a similarity, performing MSn (e.g., MS3) on the top N product ions in terms of relative abundance (e.g., only on the top two, top three, etc.), performing MSn on the top N fragments that do not correspond to a candidate mass spectrum, performing an alternative dissociation technique on the same precursor ions or the product ions, performing the same dissociation technique using different operational parameters such as different energy used for the fragmentation cell, performing a reverse polarity MS scan and perform any of the techniques described herein, or add the precursor ion that formed the product ions to an exclusion list to refrain from performing MSn in the future.

Another operation can include, upon determining a similarity, determining that the precursor ion has a particular delta mass difference from the compound of interest, or unmetabolized compound. A MS1 precursor ion scan can be performed again, but with different operational parameters (e.g., different resolution, offset isolation, etc.) to identify if there is more than one precursor ion in the isolation window. For example, if the delta mass difference is 15 m/z (e.g., representing a M + NH2 group), a higher resolution MS1 scan can be subsequently performed to capture the C13 isotope. This allows for a determining of there is more than one peak, possibly indicative of the addition of OH (M + 16).

While some of the examples described herein involve a metabolite that is the result of biological metabolism, other types of metabolites can also be identified using the techniques described herein. For example, a xenobiotic in water may be modified by ultraviolet (UV) light, resulting the xenobiotic falling apart and getting oxidized. The techniques described can identify the pieces as long as the mass spectra are similar to the xenobiotic. In another example, modified peptides can be identified in a similar manner.

In some implementations, the mass spectrum of an unmetabolized compound is provided in the mass spectra library. However, additional mass spectra can be predicted and then inserted into the mass spectra library. For example, predicted metabolites of a compound can be predicted in silico and added to the mass spectra library.

The examples describe techniques for the RTS for a metabolite, however, other biomolecules can be identified and the mass spectrometer can perform a specific action upon the identification. For example, in addition to proteins and their peptides, other types of biomolecules that can be used with the techniques include lipids, nucleic acids, metabolites, oligosaccharides, polysaccharides, and the like. Moreover, other large molecules other than biomolecules can be identified, in addition to small molecules. Thus, the experimental mass spectrum can be generated for many different types of molecules, the database can store information related to possible candidates, the RTS can be performed to identify a candidate, and MS2 or MS3 or other operations can be performed based on the capabilities of the mass spectrometer.

The tandem mass spectrometers described in the examples can be triple quadrupole mass spectrometers (QqQ), quadrupole time-of-flight mass spectrometers (QqTOF), or other types of mass spectrometers. Additionally, while the examples describe tandem mass spectrometry in space, tandem mass spectrometry in time can also be used with the techniques described herein. In a tandem mass spectrometer in time, a single mass analyzer can be used. Moreover, more than two mass analyzers can be disposed within the mass analyzer, as also discussed with the example of Figure 5.

The databases described in the examples are stored locally with the controller system of the mass spectrometer. However, cloud-based implementations can also be used in which the databases are stored on a remote server that is accessible by the controller. Additionally, hybrid approaches can be implemented with the RTS techniques. For example, a smaller database stored in the system of the mass spectrometer can be searched in parallel with a larger database stored in a remote server. A hybrid approach can allow for a smaller dataset that includes higher likelihood candidates to be identified relatively quickly. If the compound under analysis is not identified with the local database, the remote database can search a larger dataset to attempt to identify a candidate.

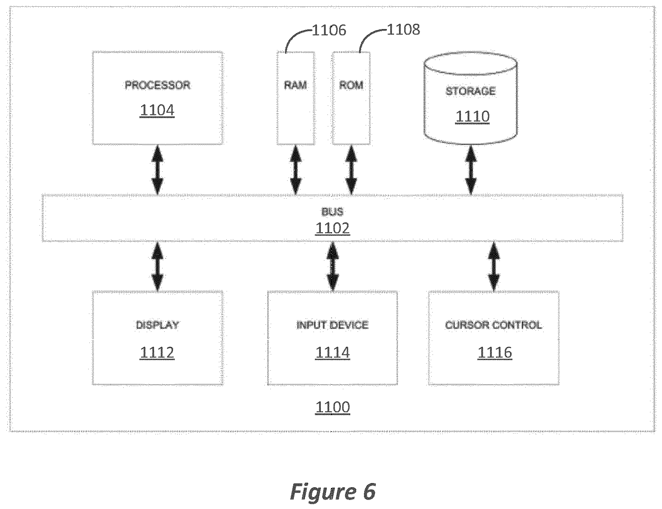

Figure 6 illustrates an example of an electronic device which may be used to implement some of the implementations. The electronic device of Figure 6 can store or use a computer program product including one or more non-transitory computer-readable media having computer programs instructed stored therein, the computer program instructions being configured such that, when executed by one or more computing devices, the computer program instructions cause the one or more computing devices to implement the functionalities described in the examples.

In Figure 6, computer system 1100 can implement any of the methods or techniques described herein. For example, computer system 1100 can implement controller circuit 115 in Figure 1. Thus, the operation of components of the associated mass spectrometer may be adjusted in accordance with calculations or determinations made by computer system 1100. In various embodiments, computer system 1100 can include a bus 1102 or other communication mechanism for communicating information, and a processor 1104 coupled with bus 1102 for processing information. In various embodiments, computer system 1100 can also include a memory 1106, which can be a random-access memory (RAM) or other dynamic storage device, coupled to bus 1102, and instructions to be executed by processor 1104. Memory 1106 also can be used for storing temporary variables or other intermediate information during execution of instructions to be executed by processor 1104. In various embodiments, computer system 1100 can further include a read only memory (ROM) 1108 or other static storage device coupled to bus 1102 for storing static information and instructions for processor 1104. A storage device 1110, such as a magnetic disk or optical disk, can be provided and coupled to bus 1102 for storing information and instructions.

In various embodiments, computer system 1100 can be coupled via bus 1102 to a display 1112, such as a cathode ray tube (CRT) or liquid crystal display (LCD), for displaying information to a computer user. An input device 1114, including alphanumeric and other keys, can be coupled to bus 1102 for communicating information and command selections to processor 1104. Another type of user input device is a cursor control 1116, such as a mouse, a trackball or cursor direction keys for communicating direction information and command selections to processor 1104 and for controlling cursor movement on display 1112. This input device typically has two degrees of freedom in two axes, a first axis (i.e., x) and a second axis (i.e., y), that allows the device to specify positions in a plane.

A computer system 1100 can perform the techniques described herein. Consistent with certain implementations, results can be provided by computer system 1100 in response to processor 1104 executing one or more sequences of one or more instructions contained in memory 1106. Such instructions can be read into memory 1106 from another computer-readable medium, such as storage device 1110. Execution of the sequences of instructions contained in memory 1106 can cause processor 1104 to perform the processes described herein. In various embodiments, instructions in the memory can sequence the use of various combinations of logic gates available within the processor to perform the processes describe herein. Alternatively hard-wired circuitry can be used in place of or in combination with software instructions to implement the present teachings. In various embodiments, the hard-wired circuitry can include the necessary logic gates, operated in the necessary sequence to perform the processes described herein. Thus implementations described herein are not limited to any specific combination of hardware circuitry and software.

The term "computer-readable medium" as used herein refers to any media that participates in providing instructions to processor 1104 for execution. Such a medium can take many forms, including but not limited to, non-volatile media, volatile media, and transmission media. Examples of non-volatile media can include, but are not limited to, optical or magnetic disks, such as storage device 1110. Examples of volatile media can include, but are not limited to, dynamic memory, such as memory 1106. Examples of transmission media can include, but are not limited to, coaxial cables, copper wire, and fiber optics, including the wires that comprise bus 1102.

Common forms of non-transitory computer-readable media include, for example, a floppy disk, a flexible disk, hard disk, magnetic tape, or any other magnetic medium, a CD-ROM, any other optical medium, punch cards, paper tape, any other physical medium with patterns of holes, a RAM, PROM, and EPROM, a FLASH-EPROM, any other memory chip or cartridge, or any other tangible medium from which a computer can read.

In accordance with various embodiments, instructions configured to be executed by a processor to perform a method are stored on a computer-readable medium. The computer-readable medium can be a device that stores digital information. For example, a computer-readable medium includes a compact disc read-only memory (CD-ROM) as is known in the art for storing software. The computer-readable medium is accessed by a processor suitable for executing instructions configured to be executed.

In various embodiments, the methods of the present teachings may be implemented in a software program and applications written in conventional programming languages such as C, C++, etc.

While the techniques are described in conjunction with various implementations or embodiments, it is not intended that the techniques be limited to such embodiments. On the contrary, the techniques encompass various alternatives, modifications, and equivalents, as will be appreciated by those of skill in the art.

Further, in describing various embodiments, the specification may have presented a method and/or process as a particular sequence of steps. However, to the extent that the method or process does not rely on the particular order of steps set forth herein, the method or process should not be limited to the particular sequence of steps described. As one of ordinary skill in the art would appreciate, other sequences of steps may be possible. Therefore, the particular order of the steps set forth in the specification should not be construed as limitations on the claims. In addition, the claims directed to the method and/or process should not be limited to the performance of their steps in the order written, and one skilled in the art can readily appreciate that the sequences may be varied and still remain within the spirit and scope of the various embodiments.

The embodiments described herein, can be practiced with other computer system configurations including hand-held devices, microprocessor systems, microprocessor-based or programmable consumer electronics, minicomputers, mainframe computers and the like. The embodiments can also be practiced in distributing computing environments where tasks are performed by remote processing devices that are linked through a network.

It should also be understood that the embodiments described herein can employ various computer-implemented operations involving data stored in computer systems. These operations are those requiring physical manipulation of physical quantities. Usually, though not necessarily, these quantities take the form of electrical or magnetic signals capable of being stored, transferred, combined, compared, and otherwise manipulated. Further, the manipulations performed are often referred to in terms, such as producing, identifying, determining, or comparing.

Any of the operations that form part of the embodiments described herein are useful machine operations. The embodiments, described herein, also relate to a device or an apparatus for performing these operations. The systems and methods described herein can be specially constructed for the required purposes or it may be a general purpose computer selectively activated or configured by a computer program stored in the computer. In particular, various general purpose machines may be used with computer programs written in accordance with the teachings herein, or it may be more convenient to construct a more specialized apparatus to perform the required operations.

Certain embodiments can also be embodied as computer readable code on a computer readable medium. The computer readable medium is any data storage device that can store data, which can thereafter be read by a computer system. Examples of the computer readable medium include hard drives, network attached storage (NAS), read-only memory, random-access memory, CD-ROMs, CD-Rs, CD-RWs, magnetic tapes, and other optical and non-optical data storage devices. The computer readable medium can also be distributed over a network coupled computer systems so that the computer readable code is stored and executed in a distributed fashion.

## Claims

1. A method of operating a mass spectrometer to analyze a metabolite of an unmetabolized compound, comprising:
introducing precursor ions generated from a sample into the mass spectrometer during an introduction period;
fragmenting the precursor ions to form product ions;
acquiring a first mass spectrum representative of the product ions;
generating, during the introduction period, scores indicative of a similarity between the first mass spectrum and a candidate mass spectrum of a candidate compound, the candidate mass spectrum stored in a mass spectra database, the scores indicating that the first mass spectrum represents a metabolite of the candidate compound; and
performing an action on the precursor ions or the product ions based on a distribution of the scores.

2. The method of claim 1, wherein the action includes performing MS3 on the product ions.

3. The method of claim 1, wherein the action includes adjusting operational parameters of a fragmentation cell and fragmenting the precursor ions or the product ions with the fragmentation cell using the operational parameters.

4. The method of claim 1, wherein the unmetabolized compound is a pharmaceutical drug.

5. A method of performing mass spectrometry, comprising:
introducing precursor ions generated from a sample into the mass spectrometer during an introduction period;
fragmenting the precursor ions to form product ions;
acquiring a first mass spectrum representative of the product ions;
generating, during the introduction period, scores indicative of a similarity between the first mass spectrum and a candidate mass spectrum of a candidate compound; and
performing an action on the precursor ions or the product ions based on a distribution of the scores.

6. The method of claim 5, wherein the scores indicate that the first mass spectrum is a metabolite of the candidate compound.

7. The method of claim 6, wherein the candidate compound is a pharmaceutical drug.

8. The method of claim 5, wherein the action includes performing MS3 on the product ions.

9. The method of claim 5, wherein the action includes adjusting operational parameters of a fragmentation cell and fragmenting the precursor ions or the product ions with the fragmentation cell using the operational parameters.

10. The method of claim 5, wherein the action includes one or more of: (a) performing MSn on a top N product ions in terms of relative abundance, (b) performing MSn on the top N fragments that do not correspond to the candidate mass spectrum,(c) performing an alternative dissociation technique on the same precursor ions or the product ions, (d) performing the same dissociation technique using different energy for the fragmentation cell, (e) performing a reverse polarity MS scan, or (f) adding the precursor ion that formed the product ions to an exclusion list to refrain from performing MSn in the future.

11. The method of claim 5, wherein the scores include a cosine score and a confidence score related to a quality of the similarity.

12. A mass spectrometry system operable to analyze a metabolite of an unmetabolized compound, comprising:
an ion source, the ion source introducing precursor ions generated from a sample into the mass spectrometry system during an introduction period;
a mass analyzer separating the ions by mass-to-charge ratio;
a detector detecting the separated ions from the mass analyzer and generating a representative signal; and
a controller generating a first mass spectrum representative of the ions based on the representative signal from the detector and generating, during the introduction period, scores indicative of a similarity between the first mass spectrum and a candidate mass spectrum of a candidate compound.

13. The system of claim 12, wherein the scores indicate that the first mass spectrum is a metabolite of the candidate compound.

14. The system of claim 12, wherein the candidate compound is a pharmaceutical drug.

15. The system of claim 12, wherein the ions received from the ion source are precursor ions and the system further comprises a fragmentation cell receiving the precursor ions from the ion source and fragmenting the precursor ions to form product ions.

16. The system of claim 15 wherein the controller further causes an action to be performed on the ions based on a distribution of the scores.

17. The system of claim 16, wherein the action includes adjusting operational parameters of a fragmentation cell and fragment the precursor ions or the product ions with the fragmentation cell using the operational parameters.

18. The system of claim 16, wherein the action includes performing MS3 on the product ions.

19. The system of claim 15, wherein the action includes one or more of: (a) performing MSn on a top N product ions in terms of relative abundance, (b) performing MSn on the top N fragments that do not correspond to the candidate mass spectrum,(c) performing an alternative dissociation technique on the same precursor ions or the product ions, (d) performing the same dissociation technique using different energy for the fragmentation cell, (e) performing a reverse polarity MS scan, or (f) adding the precursor ion that formed the product ions to an exclusion list to refrain from performing MSn in the future.

20. A mass spectrometry system operable to analyze a metabolite of an unmetabolized compound, comprising:
a controller;
a computer program product including one or more non-transitory computer-readable media having computer program instructions stored therein, the computer program instructions being configured such that, when executed by the controller, the computer program instructions cause the mass spectrometry system to perform a set of acts comprising:
introducing precursor ions generated from a sample into the mass spectrometer during an introduction period;
fragmenting the precursor ions to form product ions;
acquiring a first mass spectrum representative of the product ions;
generating, during the introduction period, scores indicative of a similarity between the first mass spectrum and a candidate mass spectrum of a candidate compound; and
performing an action on the precursor ions or the product ions based on a distribution of the scores.
